# EUROPEAN PATENT SPECIFICATION

(11) **EP 0 415 126 B1**
(45) Date of publication and mention of the grant of the patent: **01.02.1995**
(21) Application number: 90114999.7
(22) Date of filing: 04.08.1990
(51) Int. Cl.: A61K 7/16, A61K 7/26

(54) **Anti-dental plaque agents**
Zahnbelag verhindernde Mittel
Agents prévenants la plaque dentaire

(30) Priority: 30.08.1989 JP 221816/89
(43) Date of publication of application: 06.03.1991
(73) Proprietor: MITSUI NORIN CO., LTD., Tokyo (JP)
(72) Inventor: Hara, Yukihiko, Fujieda-shi, Shizuoka-ken (JP); Hattori, Masao, Kosugi-cho, Imizu-gun, Toyama-ken (JP)
(74) Representative: Türk, Dietmar

(56) References cited:
- PATENT ABSTRACTS OF JAPAN, unexamined applications, C section, vol. 7, no. 46,February 23, 1983 THE PATENT OFFICE JAPANESE GOVERNMENT page 26 C 153
- PATENT ABSTRACTS OF JAPAN, unexamined applications, C section, vol. 10, no.280, September 24, 1986 THE PATENT OFFICE JAPANESE GOVERNMENT page 54 C 374

## Description

### BACKGROUND OF THE INVENTION

The present invention relates to the use of tea polyphenol compounds for preventing dental plaque and, more particularly, for preparing an anti-dental plaque agent which inhibits the formation of water-insoluble glucan by glucosyltransferase (hereinafter often abbreviated as GTF) which Streptococcus mutans produces extracellularly.

Dental caries is an infectious disease of the hard tissue of teeth which has afflicted mankind from ancient times up to now and is common all over the world.

Of pathogenic bacteria causing dental caries, Streptococcus mutans is a primary important cariogenic bacterium. Glucan which is a polymer of glucose is synthesized from sucrose by the action of glucosyltransferase which is produced extracellularly by one above pathogenic bacteria. Water-insoluble glucan has the property of adhering firmly to a smooth tooth surface. This water-insoluble glucan and Streptococcus mutans adhering to the tooth surface through it continue to grow and eventually overspread the tooth surface. This is called "dental plaque". Making use of various sugars, Streptococcus mutans generates some acids inclusive of lactic acid in the dental plaque, which in turn decalcify enamel. At this time, water-insoluble glucan is considered to play a role in preventing the diffusion of the acids.

Thus, the primary action of Streptococcus mutans in cariogenesis is to form water-insoluble glucan by GTF.

Heretofore, various pharmaceuticals for inhibiting cariogenesis have been proposed. However, there is still strong demand toward developing a pharmaceutical preparation which is so harmless to the human body that it can be safe in use.

Japanese patent application No. 56-81892 describes that a toothpaste composition having unique taste can be provided by grinding the leaves of black tea to extremely fine powder and adding the powder to the raw-materials of the toothpaste. For such purpose the leaves of black tea are ground in the presence of water or water-containing sugars such as liquid invert sugar, sucrose, sorbitol, etc., or a polyhydric alcohol such as glycerin, propylene glycol, etc. to particles having a diameter of 1 ∼ 50 »m, and the paste is added to a toothpaste composition in an amount of preferably 0.05 ∼ 0.2 wt.-%.

In Japanese patent application No. 59-221094 an agent for dental caries for preventing dental caries is described which has the ability to inhibit activity of glycosyltransferase resulting from Streptococcus mutans, comprising a gallotannin, chebulinic acid, granatin A, etc. as active ingredients.

In EP-A-0 256 566 the use of usnic acid or derivatives thereof, especially in the dextrorotatory form in the treatment of dental caries is described. According to such disclosure the usnic acid is active as specific bacteriostatic against Streptococcus mutans which is the primary pathogenic microorganism of cariogenic lesions.

In EP-A-0 306 853 a cosmetic preparation having germicide properties is described, which contains 5-hydroxy-1,4-naphthoquinone as such in the form of parts of plants or in the form of extracts, which can be obtained from the parts of plants. Toothpaste and mouth-washing preparations, deodorants and footpowder are preferred cosmetic preparations.

As a result of intensive and extensive studies made in search of a substance having the desired pharmaceutical effect from naturally occurring materials, rather than from chemically synthesized materials, it has now been found that said substance is contained in the components of tea leaves.

As hitherto known, tea is effective against preventing dental caries. However, the active ingredient has been considered to be fluorine contained in tea, which enhances dentine per se, preventing it from being attacked by cariogenic bacteria and decalcified by acids.

The present inventor has now found that the effect of tea upon preventing cariogenesis is obtained by a material composed mainly of tea polyphenols which, separately from the action of fluorine, inhibit considerably the ability of GTF to produce water-insoluble glucan.

A principal component of tea is tea polyphenol compounds, and said tea polyphenol compounds include the tea catechin compounds represented by the general formula (I) given below and the theaflavin compounds represented by the general formula (II) given below, and also thearubigin: 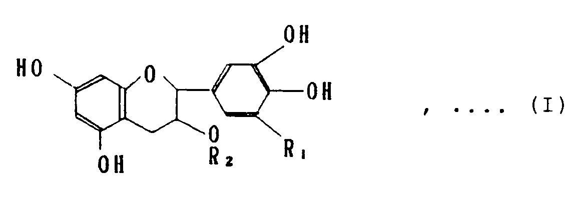
in which R₁ is a hydrogen atom or a hydroxy group and R₂ is a hydrogen atom or a 3,4,5-trihydroxy benzoyl group; and 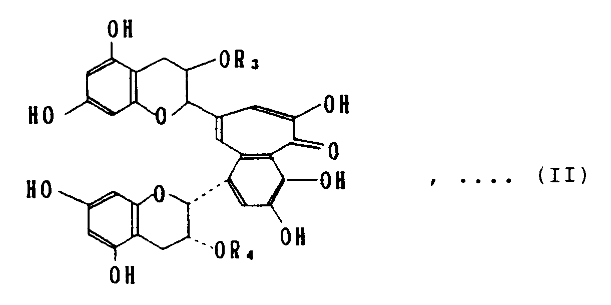
in which R₃ and R₄ are, each independently from the other, a hydrogen atom or a 3,4,5-trihydroxy benzoyl group.

Particular examples of the tea catechin compounds represented by the general formula (I) include: (-)epicatechin, which is a compound of the formula (I) with R₁ = H and R₂ = H; (-)epigallocatechin, which is a compound of the formula (I) with R₁ = OH and R₂ = H; (-)epicatechin gallate, which is a compound of the formula (I) with R₁ = H and R₂ = 3,4,5-trihydroxy benzoyl group; and (-)epigallocatechin gallate, which is a compound of the formula (I) with R₁ = OH and R₂ = 3,4,5-trihydroxy benzoyl group. Particular examples of the theaflavin compounds include; free theaflavin, which is a compound of the formula (II) with R₃ = H and R₄ = H; theaflavin monogallate A, which is a compound of the formula (II) with R₃ = 3,4,5-trihydroxy benzoyl group and R₄ = H; theaflavin monogallate B, which is a compound of the formula (II) with R₃ = H and R₄ = 3,4,5-trihydroxy benzoyl group; and theaflavin digallate, which is a compound of the formula (II) with R₃ = 3,4,5-trihydroxy benzoyl group and R₄ = 3,4,5-trihydroxy benzoyl group.

The above described tea polyphenol compounds can be prepared from tea leaves as the starting material and a method for the preparation thereof and a typical example of the product composition are described, for example, in Japanese Patent Kokai 59-219384, 60-13780 and 61-130285, etc.

The tea polyphenol compounds may be added to foodstuffs such as starch jelly, mouth wash and dentifrice by dissolving and mixing the main components or the tea polyphenols directly in and with water and alcohol. The concentration may be preferably 100 to 5,000 ppm, more preferably 200 to 1,000 ppm.

Even when added to not only pharmaceuticals but also foodstuffs, the anti-dental plaque agent of the present invention is most unlikely to cause harmful side effects to the human body, since it is chiefly composed of a naturally occurring material or materials drunk usually in large amounts. In low concentrations, the present agent is effective in inhibiting the formation of water-insoluble glucan by glucosyltransferase of Streptococcus mutans. Thus, the anti-dental plaque agent of the present invention is very effective for preventing or reducing cariogenesis.

The present invention will now be explained specifically but not exclusively with reference to the following example, in which the tea polyphenols used were a green tea extract, a composition containing 30% or more of the catechins (trade name: "Polyphenone 30" made by Mitsui Norin Co., Ltd.) or a green tea extract, a composition containing 90% or more of the catechins (trade name: "Polyphenone 100" made by Mitsui Norin Co., Ltd.) or a black tea extract, a composition containing the theaflavin (trade name: "Polyphenone TF" made by Mitsui Norin Co., Ltd.).

### Example 1

Crude glucosyltransferase was refined from Streptococcus mutans OMZ 176 according to the method described by Mukasa and Slads in Infect. Immun., 8, 555 (1973) and 9, 419 (1974).

The above GTF, labeled sucrose and the tea polyphenol compounds were mixed together to the following final concentrations, and was regulated with a 50 mM phosphate buffer (with pH 6.8).
GTF: 0.34 mg protein/ml
0.1 mM (¹⁴C) sucrose: 0.1 »Ci/»l
Present agent: 1 to 10 mg/ml or 1 to 10 mM.

Twenty (20) »l of the mixture solution were subjected to a reaction for 60 minutes at 37°C. After the completion of the reaction, 5 »l of the reaction mixture and 5 »l of supernatant obtained by centrifugation of the reaction mixture at 5,500 x g for 3 minutes were applied to a filter paper respectively, and then developed with a developing solution of butanol-pyridine-water (6:4:3), followed by counting with a scintillation counter. The amount of water-insoluble glucan was found by a difference between the measurements of the reaction mixture and the supernatant. The GTF inhibiting ability is expressed in terms of the rate of inhibiting the formation of water-insoluble glucan with respect to a control to which the tea polyphenol compounds were not added. The results are shown in Tables 1 and 2.

**Table 1**

| Effects of Tea Preparations on Water-Insoluble Glucan Formation | | |
|---|---|---|
| Sample | Concentration (mg/ml) | Inhibition (%) |
| Polyphenon 30 | 1.0 | 23 |
| | 10.0 | 87 |
| Polyphenon 100 | 1.0 | 28 |
| | 10.0 | 93 |
| Polyphenon B | 1.0 | 21 |
| | 10.0 | 91 |
| Crude theaflavins | 1.0 | 77 |
| | 10.0 | 100 |
| Polyphenon-protein complex | 1.0 | 0 |
| | 10.0 | 19 |

**Table 2**

| Effects of Tea Polyphenols on Water-Insoluble Glucan Formation | | | |
|---|---|---|---|
| Sample | Configuration | Concentration (mM) | Inhibition (%) |
| (+)-Catechin | 2R,3S | 1.0 | 22 |
| | | 10.0 | 62 |
| (-)-Catechin | 2S,3R | 1.0 | 15 |
| | | 10.0 | 45 |
| (+)-Epicatechin | 2S,3S | 1.0 | 12 |
| | | 10.0 | 42 |
| (-)-Epicatechin | 2R,3R | 1.0 | 5 |
| | | 10.0 | 42 |
| (+)-Gallocatechin | 2R,3S | 1.0 | 15 |
| | | 10.0 | 51 |
| (-)-Gallocatechin | 2S,3R | 1.0 | 20 |
| | | 10.0 | 30 |
| (-)-Epigallocatechin | 2R,3R | 1.0 | 13 |
| | | 10.0 | 25 |
| (-)-Epicatechin gallate | 2R,3R | 1.0 | 35 |
| | | 10.0 | 83 |
| (-)-Gallocatechin gallate | 2S,3R | 1.0 | 47 |
| | | 10.0 | 95 |
| (-)-Epigallocatechin gallate | 2R,3R | 1.0 | 42 |
| | | 10.0 | 75 |
| Free theaflavin | | 1.0 | 57 |
| | | 10.0 | 98 |
| Theaflavin monogallate A | | 1.0 | 64 |
| | | 10.0 | 97 |
| Theaflavin monogallate B | | 1.0 | 47 |
| | | 10.0 | 98 |
| Theaflavin digallate | | 1.0 | 56 |
| | | 10.0 | 98 |

## Claims

1. Use of at least one tea polyphenol compound of the general formula (I) and (II) 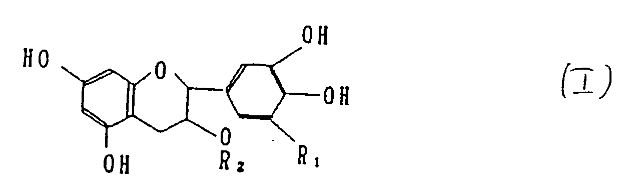 in which R₁ is a hydrogen atom or a hydroxy group and R₂ is a hydrogen atom or a 3,4,5-trihydroxy benzoyl group; and 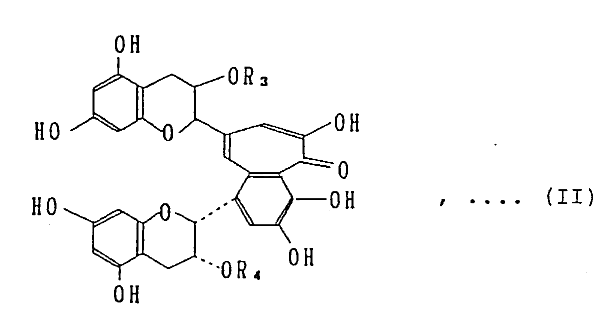 in which R₃ and R₄ are, each independently from the other, a hydrogen atom or a 3,4,5-trihydroxy benzoyl group, for the preparation of an agent for preventing dental plaque.

2. Use according to Claim 1, wherein said tea polyphenol is tea polyphenol selected from the group consisting of epigallocatechin gallate, epicatechin gallate, epigallocatechin, epicatechin, catechin, isomers thereof, free theaflavin, theaflavin monogallate A, theaflavin monogallate B and theaflavin digallate.

3. Use of the compound of the formula I or II as defined in claim 1 for the preparation of a anti-dental plaque agent for the treatment of dental plaque.

4. Use of a compound of the formula I or II as defined in claim 1 for the preparation of a medicament for reducing cariogenesis.

5. Use of at least one tea polyphenol compound of the general formula (I) and (II) 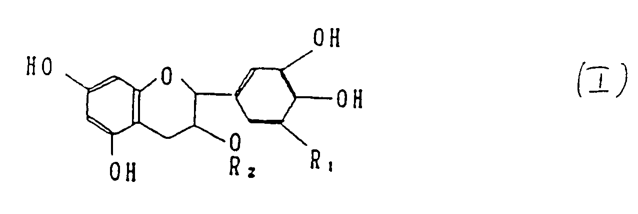 in which R₁ is a hydrogen atom or a hydroxy group and R₂ is a hydrogen atom or a 3,4,5-trihydroxy benzoyl group; and 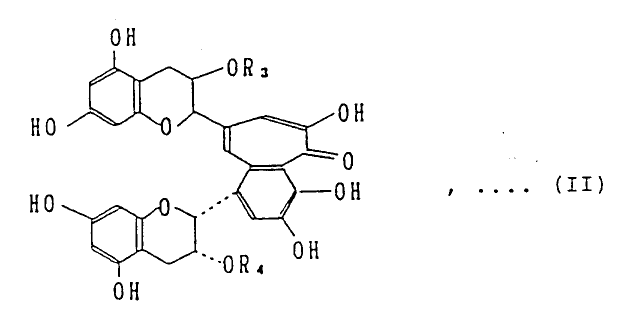 in which R₃ and R₄ are, each independently from the other, a hydrogen atom or a 3,4,5-trihydroxy benzoyl group, as an additive for foodstuff for preventing dental plaque.

## Patentansprüche

1. Verwendung von mindestens einer Tee-Polyphenol-Verbindung der allgemeinen Formel (I) und (II) 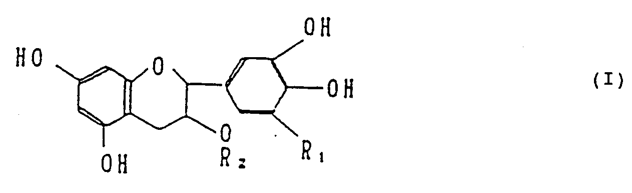 bei der R₁ ein Wasserstoffatom oder eine Hydroxygruppe ist und R₂ ein Wasserstoffatom oder eine 3,4,5-Trihydroxybenzoylgruppe ist; und 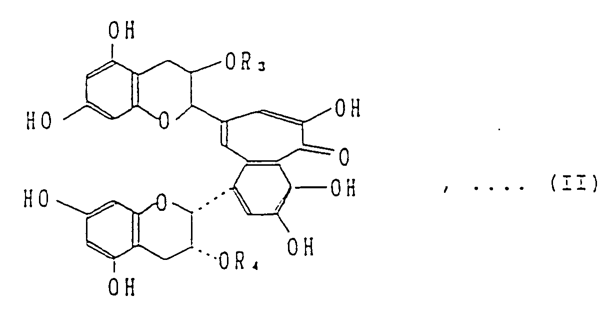 bei der R₃ und R₄ jeweils unabhängig voneinander ein Wasserstoffatom oder eine 3,4,5-Trihydroxybenzoylgruppe sind, zur Herstellung eines Mittels zur Verhütung von Zahnbelag.

2. Verwendung nach Anspruch 1, worin das Tee-Polyphenol Tee-Polyphenol ist, ausgewählt aus der aus Epigallocatechingallat, Epicatechingallat, Epigallocatechin, Epicatechin, Catechin, Isomeren davon, freiem Theaflavin, Theaflavinmanogallat A, Theaflavinmonogallat B und Theaflavindigallat bestehenden Gruppe.

3. Verwendung der Verbindung der Formel I oder II wie in Anspruch 1 definiert für die Herstellung eines Mittels gegen Zahnbelag zur Behandlung von Zahnbelag.

4. Verwendung einer Verbindung der Formel I oder II wie in Anspruch 1 definiert zur Herstellung eines Medikaments zur Reduzierung der Kariogenese.

5. Verwendung von mindestens einer Tee-Polyphenol-Verbindung der allgemeinen Formel (I) und (II) 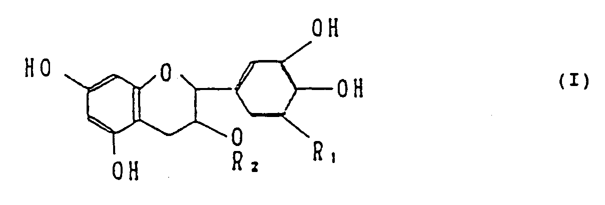 bei der R₁ ein Wasserstoffatom oder eine Hydroxygruppe ist und R₂ ein Wasserstoffatom oder eine 3,4,5-Trihydroxybenzoylgruppe ist; und 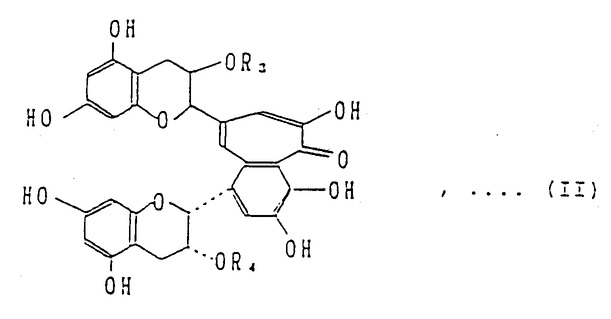 bei der R₃ und R₄ jeweils unabhängig voneinander ein Wasserstoffatom oder eine 3,4,5-Trihydroxybenzoylgruppe ist, als Zusatz für Lebensmittel zur Verhütung von Zahnbelag.

## Revendications

1. Utilisation d'au moins un polyphénol du thé de formule générale (I) ou (II) 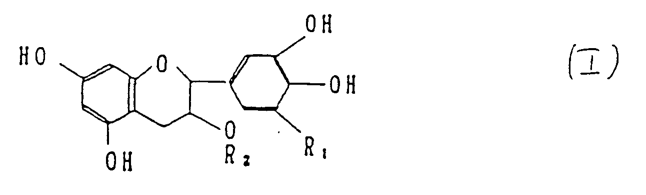 où R₁ est un atome d'hydrogène ou un groupe hydroxyle et R₂ est un atome d'hydrogène ou un groupe 3,4,5-trihydroxybenzoyle ; et 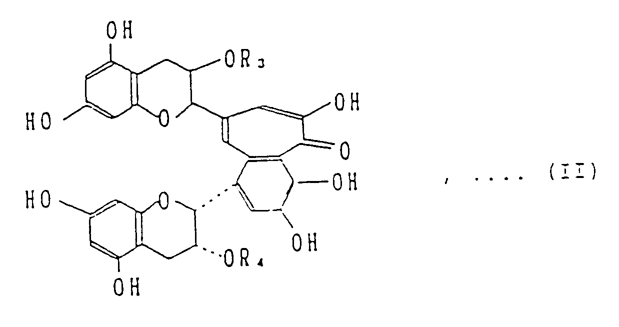 où R₃ et R₄ sont chacun, indépendamment de l'autre, un atome d'hydrogène ou un groupe 3,4,5-trihydroxybenzoyle, pour la préparation d'un agent destiné à la prévention de la plaque dentaire.

2. Utilisation selon la revendication 1, dans laquelle ledit polyphénol du thé est un polyphénol de thé choisi dans le groupe formé par le gallate d'épigallocatéchine, le gallate d'épicatéchine, l'épigallocatéchine, l'épicatéchine, la catéchine, leurs isomères, la théaflavine libre, le monogallate A de théaflavine, le monogallate B de théaflavine et le digallate de théaflavine.

3. Utilisation du composé de formule I ou II tel que défini dans la revendication 1, pour la préparation d'un agent anti-plaque dentaire destiné au traitement de la plaque dentaire.

4. Utilisation d'un composé de formule I ou II tel que défini dans la revendication 1, pour la préparation d'un médicament destiné à réduire la cariogenèse.

5. Utilisation d'au moins un polyphénol du thé de formule générale (I) ou (II) 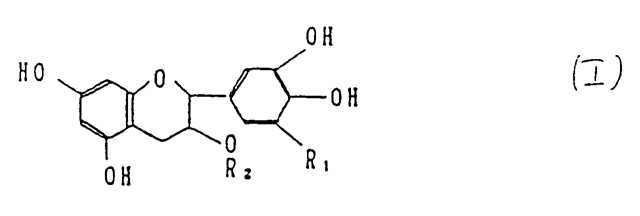 où R₁ est un atome d'hydrogène ou un groupe hydroxyle et R₂ est un atome d'hydrogène ou un groupe 3,4,5-trihydroxybenzoyle ; et 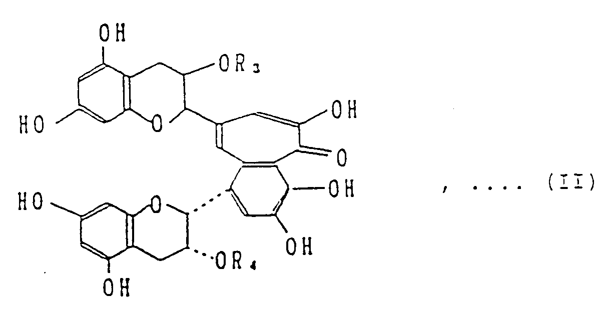 où R₃ et R₄ sont chacun, indépendamment de l'autre, un atome d'hydrogène ou un groupe 3,4,5-trihydroxybenzoyle, comme additif pour produit alimentaire destiné à la prévention de la plaque dentaire.
